# EUROPEAN PATENT APPLICATION

(11) **EP 4 767 942 A1**
(43) Date of publication of application: **01.07.2026**
(21) Application number: 24872951.9
(22) Date of filing: 26.09.2024
(51) Int. Cl.: A61B 5/327, A61B 5/00, A61B 5/28, A61B 5/257

(54) **METHOD, PROGRAM, AND DEVICE FOR ANALYZING SINGLE-LEAD ELECTROCARDIOGRAM BY USING ARTIFICIAL INTELLIGENCE**

(30) Priority: 26.09.2023 KR 20230128754
(71) Applicant: Medical AI Co., Ltd., Seoul 06180 (KR)
(72) Inventor: KWON, Joonmyoung, Seoul 06180 (KR)
(74) Representative: Patentanwaltskanzlei Matschnig & Forsthuber OG
(86) International application number: PCT/KR2024/014576
(87) International publication number: WO 2025/071243

(57) **Abstract**

In accordance with an embodiment of the present disclosure, a prediction method, a program, and a device for analyzing a single-lead electrocardiogram using artificial intelligence, performed by a computing device, are provided. The method may comprise: acquiring single-lead electrocardiogram data; and processing the acquired single-lead electrocardiogram data by analyzing at least one of input conditions of a plurality of pre-trained neural network models for reading single-lead electrocardiogram data, based on a length of the acquired single-lead electrocardiogram data.

## Description

### TECHNICAL FIELD

The present disclosure relates to artificial intelligence technology in the medical field, and more specifically, to a method for analyzing a single-lead electrocardiogram (hereinafter, "ECG") using artificial intelligence to diagnose various diseases and health conditions of a person whose single-lead ECG has been measured.

### BACKGROUND OF THE INVENTION

An ECG test is an examination that measures electrical signals generated by the heart and is widely used for diagnosing heart diseases and the like. An ECG measured in a medical institution such as a hospital is generated as a total of 12 leads by attaching four electrodes to the arms and legs and six electrodes to the chest, and recording the heart's signals measured from different directions.

Measuring a 12-lead ECG is difficult to perform for an extended period because it requires attaching electrodes all over the body. A 12-lead ECG is typically recorded for about 10 seconds. Therefore, in the case of a disease with intermittent symptoms, such as arrhythmia, it is not easy to detect by interpreting a 12-lead ECG measured over a short period.

To diagnose a disease with intermittent symptoms, such as arrhythmia, a method of long-term ECG measurement using a patch-type measuring device is used, rather than the method of attaching electrodes to the arms, legs, and chest. This measurement method typically involves attaching an electrode only to the chest, and therefore, only a single-lead ECG is recorded.

Meanwhile, a single-lead ECG has the advantage of being easily measurable in daily life through portable measuring devices. Accordingly, there is a growing trend of attempting to diagnose not only diseases with intermittent symptoms like arrhythmia but also various other diseases and health conditions using a single-lead ECG. In particular, attempts are being made to integrally diagnose various diseases and health conditions through healthcare platforms. However, since the type of ECG data required for diagnosis can vary depending on the disease or health condition to be analyzed, it may be difficult to accurately diagnose various diseases or health conditions by using the single-lead ECG measured from a device as is.

### SUMMARY OF THE INVENTION

### TECHNICAL PROBLEM

An object of the present disclosure is to provide a method for integrally diagnosing various diseases such as arrhythmia and heart failure, and health conditions such as sleep disorders, based on a single-lead ECG using artificial intelligence.

However, the problems to be solved by the present disclosure are not limited to the aforementioned problems, and other unmentioned problems can be clearly understood from the following description.

### TECHNICAL SOLUTION

In accordance with an embodiment of the present disclosure for achieving the above objects, a method for analyzing a single-lead ECG using artificial intelligence, performed by a computing device, is provided. The method may comprise: acquiring single-lead ECG data; and processing the acquired single-lead ECG data by analyzing at least one of input conditions of a plurality of pre-trained neural network models for reading single-lead ECG data, based on a length of the acquired single-lead ECG data.

Alternatively, the acquiring of the single-lead ECG data may comprise acquiring, in real time, the single-lead ECG data being measured by an ECG measurement device, or acquiring the single-lead ECG data having a predetermined length, measurement of which is completed by the ECG measurement device.

Alternatively, the processing of the acquired single-lead ECG data may comprise: when the single-lead ECG data being measured by the ECG measurement device is acquired in real time, analyzing whether a length of time-serially accumulated single-lead ECG data satisfies at least one of the input conditions; and extracting, from the time-serially accumulated single-lead ECG data, the single-lead ECG data of a length that satisfies the at least one of the input conditions.

Alternatively, the processing of the acquired single-lead ECG data may comprise: when the single-lead ECG data having the predetermined length, measurement of which is completed by the ECG measurement device, is acquired, identifying, in the single-lead ECG data having the predetermined length, an interval that satisfies at least one of the input conditions; and extracting, from the single-lead ECG data having the predetermined length, the single-lead ECG data of the interval that satisfies the at least one of the input conditions.

Alternatively, the method may further comprise determining whether to input the processed single-lead ECG data to at least one of the plurality of neural network models, by analyzing a noise level of the processed single-lead ECG data.

Alternatively, the determining of whether to input the processed single-lead ECG data to the at least one of the plurality of neural network models may comprise: analyzing whether the noise level of the processed single-lead ECG data satisfies a noise condition of a model, among the plurality of models, that has an input condition corresponding to the length of the processed single-lead ECG data; and determining whether to input the processed single-lead ECG data to the model having the input condition corresponding to the length of the processed single-lead ECG data, based on whether the noise condition is satisfied.

Alternatively, a matter regarding the length of the ECG data included in the input condition may vary depending on an analysis task of the plurality of neural network models.

Alternatively, an input condition of a model for analyzing a disease related to an ECG rhythm characteristic, among the plurality of neural network models, may relate to whether the length of the single-lead ECG data corresponds to a first time period.

Alternatively, an input condition of a model for analyzing a disease related to a characteristic other than the ECG rhythm characteristic, among the plurality of neural network models, may relate to whether the length of the single-lead ECG data corresponds to a second time period.

Alternatively, an input condition of a model for analyzing sleep, among the plurality of neural network models, may relate to whether the length of the single-lead ECG data corresponds to a third time period.

Alternatively, an input condition of a model for analyzing stress, among the plurality of neural network models, may relate to whether a minimum length of the single-lead ECG data corresponds to a fourth time period. The first time period, the second time period, the third time period, and the fourth time period may be different from each other.

Alternatively, the processing of the acquired single-lead ECG data may comprise, when the length of the acquired single-lead ECG data satisfies the input condition of the model for analyzing stress among the plurality of neural network models, converting the extracted single-lead ECG data into heart rate variability data.

Alternatively, the method may comprise generating a reading result regarding a disease or a health condition, by inputting the processed single-lead ECG data to a model, among the plurality of models, that has an input condition corresponding to the length of the processed single-lead ECG data.

In accordance with an embodiment of the present disclosure for achieving the above objects, a computer program stored on a computer-readable storage medium is provided. The computer program, when executed by one or more processors, causes operations to be performed for analyzing a single-lead ECG using artificial intelligence. The operations may comprise: acquiring single-lead ECG data; and processing the acquired single-lead ECG data by analyzing at least one of input conditions of a plurality of pre-trained neural network models for reading single-lead ECG data, based on a length of the acquired single-lead ECG data.

In accordance with an embodiment of the present disclosure for achieving the above objects, a computing device for analyzing a single-lead ECG using artificial intelligence is provided. The device may comprise: a processor comprising at least one core; a memory comprising program codes executable by the processor; and a network unit for acquiring single-lead ECG data. The processor may be configured to process the acquired single-lead ECG data by analyzing at least one of input conditions of a plurality of pre-trained neural network models for reading single-lead ECG data, based on a length of the acquired single-lead ECG data.

### ADVANTAGEOUS EFFECTS

The present disclosure may establish an integrated diagnosis system capable of diagnosing not only diseases suitable for single-lead ECG analysis but also diseases suitable for analysis of a conventional 12-lead ECG, and health conditions such as sleep disorders, by processing a single-lead ECG to match various data formats required for the analysis of various diseases and health conditions.

### BRIEF DESCRIPTION OF THE DRAWINGS

FIG. 1 is a block diagram of a computing device according to an embodiment of the present disclosure.
FIG. 2 is a block diagram illustrating an operational process of a computing device according to an embodiment of the present disclosure.
FIG. 3 is a flowchart illustrating a process of analyzing a single-lead ECG according to an embodiment of the present disclosure.
FIG. 4 is a flowchart illustrating a method for analyzing a single-lead ECG using artificial intelligence according to an embodiment of the present disclosure.

### DETAILED DESCRIPTION OF THE EMBODIMENTS

Hereinafter, embodiments of the present disclosure will be described in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and should not be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" should be understood to mean any of the natural inclusive permutations. For example, if "X uses A or B" is used in the present disclosure, unless otherwise specified or clear from the context, it may be interpreted as any one of the cases where X uses A, X uses B, or X uses both A and B.

The term "and/or" as used in the present disclosure should be understood to refer to and include all possible combinations of one or more of the associated listed concepts.

The terms "comprise" and/or "comprising" as used in the present disclosure should be understood to mean the presence of a specific feature and/or component. However, the terms "comprise" and/or "comprising" should be understood as not precluding the presence or addition of one or more other features, other components, and/or combinations thereof.

In the present disclosure, unless otherwise specified or clear from the context to indicate a singular form, the singular generally should be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data through a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented through software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within a scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have a problem-solving ability by optimizing a parameter that connects a node or a neuron through learning. A neural network "model" may include a single neural network or a set of neural networks in which a plurality of neural networks are combined.

The above description of terms is for the purpose of facilitating the understanding of the present disclosure. Therefore, it should be noted that if the above terms are not explicitly stated as limiting the content of the present disclosure, they are not used in a sense that limits the technical spirit of the content of the present disclosure.

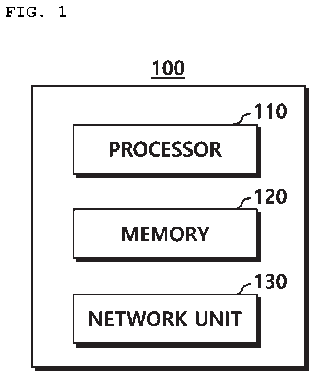

FIG. 1 is a block diagram of a computing device according to an embodiment of the present disclosure.

A computing device (100) according to an embodiment of the present disclosure may be a hardware device or a part of a hardware device that performs comprehensive processing and calculation of data, or it may be a software-based computing environment connected via a communication network. For example, the computing device (100) may be a server, which is a subject that performs intensive data processing functions and shares resources, or it may be a client that shares resources through interaction with a server. In addition, the computing device (100) may be a cloud system in which a plurality of servers and clients interact to process data comprehensively. The above description is only one example related to the type of the computing device (100), and therefore, the type of the computing device (100) may be variously configured within a scope understandable by a person of ordinary skill in the art based on the content of the present disclosure.

Referring to FIG. 1, the computing device (100) according to an embodiment of the present disclosure may comprise a processor (110), a memory (120), and a network unit (130). However, FIG. 1 is only an example, and the computing device (100) may include other components to implement a computing environment. In addition, only some of the disclosed components may be included in the computing device (100).

The processor (110) according to an embodiment of the present disclosure may be understood as a component unit including hardware and/or software for performing computing operations. For example, the processor (110) may read a computer program and perform data processing for machine learning. The processor (110) may process computational processes such as feature extraction for machine learning, error calculation based on backpropagation, and the like. The processor (110) for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor (110) described above are only examples, and the type of the processor (110) may be variously configured within a scope understandable by a person of ordinary skill in the art based on the content of the present disclosure.

The processor (110) may perform preprocessing on single-lead ECG data in consideration of the input conditions of neural network models for ECG reading. Since the input conditions of the neural network models can differ depending on the type of reading, it is necessary for the processor (110) to process the acquired data to match each of the input conditions. Therefore, the processor (110) may determine whether the single-lead ECG data satisfies at least one of the input conditions, and may process the data to match the satisfied condition. For example, when the single-lead ECG data is acquired and accumulated in real time, the processor (110) may analyze the length of the accumulated data to determine whether it satisfies at least one of the input conditions of the plurality of neural network models. If there is a satisfied input condition, the processor (110) may extract data matching the satisfied condition from the accumulated data. When the single-lead ECG data is acquired with a predetermined length, the processor (110) may extract the data to match the input conditions of the plurality of neural network models. Since the data already has a predetermined length, the processor (110) may extract data matching the length required by each of the input conditions from within the predetermined length. Through such preprocessing, the processor (110) may efficiently integrate and manage a plurality of neural network models that perform different readings.

The processor (110) may input the preprocessed single-lead ECG data to at least one of the neural network models for ECG reading to analyze the disease or health condition of a subject from whom the single-lead ECG data was measured. The processor (110) may input the data processed to match a specific input condition to a neural network model that requires the specific input condition, thereby generating a reading result regarding the disease or health condition. When data satisfying all or some of the input conditions of the plurality of neural network models is generated through preprocessing, the processor (110) may input the preprocessed data to the models corresponding to the input conditions to generate reading results. For example, if single-lead ECG data is processed to match input conditions A and B among A, B, and C, the processor (110) may input the data A processed to match input condition A to a model A that requires input condition A, thereby generating a reading result A. The processor (110) may input the data B processed to match input condition B to a model B that requires input condition B, thereby generating a reading result B. Since the single-lead ECG data was acquired but did not have a length satisfying input condition C, preprocessing was not performed, so the processor (110) may not perform a reading through model C. If preprocessing is performed to satisfy input condition C during the process of accumulating single-lead ECG data, the processor (110) may perform a reading through model C.

The processor (110) may perform post-processing on the reading results generated from all or some of the plurality of neural network models. When a plurality of reading data are generated through the aforementioned readings, the processor (110) may use a statistical technique to combine the individual result values included in the plurality of reading data to generate an integrated diagnosis result. The processor (110) may also input the individual result values included in the plurality of reading data to a post-processing neural network model to generate an integrated diagnosis result regarding a disease or health condition. For example, if outputs of an arrhythmia analysis model, a heart failure analysis model, and a stress analysis model, among the plurality of neural network models, are generated, the processor (110) may input the probability values corresponding to the output of each model to a post-processing neural network model to generate a comprehensive diagnosis result of the subject from whom the single-lead ECG data was measured. The post-processing neural network model may comprehensively analyze input values and evaluation metrics such as the output reliability of each model to generate a comprehensive reading result indicating what disease the subject from whom the ECG was measured has or what their health condition is.

Meanwhile, the neural network models for ECG reading may be pre-trained based on training data composed of single-lead ECGs. In this case, the neural network models for ECG reading may be configured to read different types of diseases or health conditions. The input conditions of the neural network models may be the same or different depending on the analysis task. For example, a model for predicting heart failure may receive training data composed of 10-second-long single-lead ECGs, extract features from the input training data, and calculate a probability value for the occurrence of heart failure. A model for predicting arrhythmia may receive training data composed of 24-hour-long single-lead ECGs, extract features from the input training data, and calculate a probability value for the occurrence of arrhythmia. The probability value calculated in each individual model may be compared with a label included in the training data input to the individual model. A loss function may be used to calculate an error in the comparison process. The parameters constituting the neural network of each model may be updated based on the error calculated through the loss function. The training of the models may be performed through this update process. In addition to the aforementioned training method, the neural network models for ECG reading of the present disclosure may be trained through methods such as unsupervised learning and self-supervised learning, depending on the structure of the model.

A memory (120) according to an embodiment of the present disclosure may be understood as a constituent unit including hardware and/or software for storing and managing data processed in a computing device (100). That is, the memory (120) may store any form of data generated or determined by the processor (110) and any form of data received by the network unit (130). For example, the memory (120) may include at least one storage medium of a flash memory type, a hard disk type, a multimedia card micro type, a card type memory, a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. In addition, the memory (120) may include a database system that controls and manages data in a predetermined system. The foregoing types of the memory (120) are only one example, and the type of the memory (120) may be variously configured within a scope understandable by a person of ordinary skill in the art based on the content of the present disclosure.

The memory (120) may structure and organize data, a combination of data, and program codes executable by the processor (110) that are necessary for the processor (110) to perform computations. For example, the memory (120) may store the ECG data received through the network unit (130) to be described later. The memory (120) may store program codes that cause a machine learning model to perform training by receiving ECG data, program codes that cause a machine learning model to perform inference according to the intended use of the computing device (100) by receiving ECG data, and processed data generated when the program codes are executed.

A network unit (130) according to an embodiment of the present disclosure may be understood as a component unit that transmits and receives data through any form of known wired and wireless communication system. For example, the network unit (130) may perform data transmission and reception using a wired or wireless communication system such as a local area network (LAN), wideband code division multiple access (WCDMA), long term evolution (LTE), wireless broadband internet (WiBro), 5th generation mobile communication (5G), ultra wide-band, ZigBee, radio frequency (RF) communication, wireless LAN, wireless fidelity (Wi-Fi), near field communication (NFC), or Bluetooth. The aforementioned communication systems are only examples, and therefore, the wired and wireless communication system for data transmission and reception of the network unit (130) may be variously applied in addition to the examples described above.

The network unit (130) may receive data necessary for the processor (110) to perform computations, through wired or wireless communication with any system or any client. In addition, the network unit (130) may transmit data generated through the computations of the processor (110), through wired or wireless communication with any system or any client. For example, the network unit (130) may receive ECG data through communication with a cloud server that performs tasks such as standardization of medical data, a database in a hospital environment, a client such as a smart watch, or a medical computing device. The network unit (130) may transmit output data of a machine learning model, intermediate data derived during the computation process of the processor (110), and processed data, through communication with the aforementioned database, server, client, or computing device.

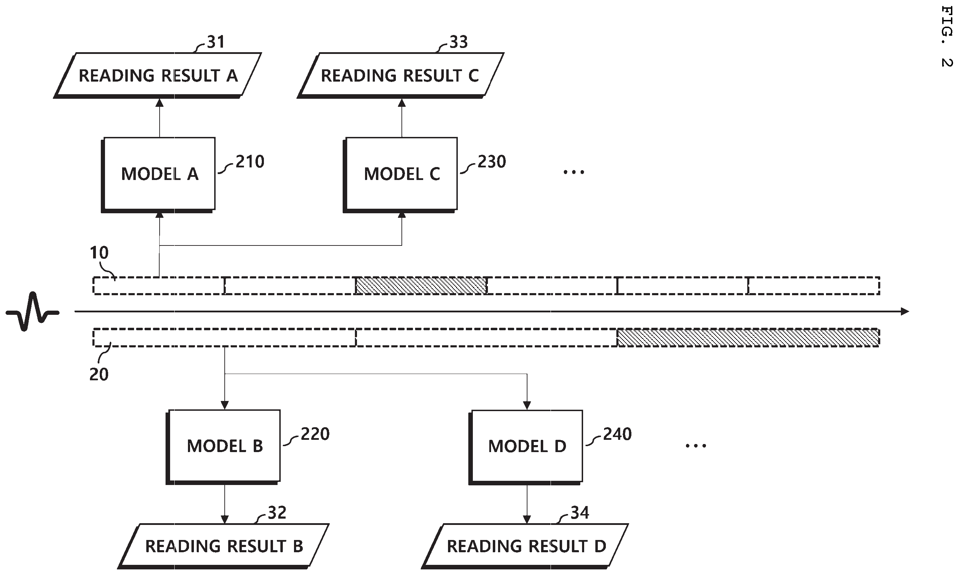

FIG. 2 is a block diagram illustrating an operational process of a computing device according to an embodiment of the present disclosure.

When single-lead ECG data is acquired, a computing device (100) according to an embodiment of the present disclosure may perform processing on the acquired data by analyzing the length of the acquired data and the input conditions of neural network models for ECG reading. When single-lead ECG data being measured by an ECG measurement device is acquired in real time, the computing device (100) may continuously store the acquired data and analyze the accumulated length of the acquired data in real time to perform processing. When single-lead ECG data having a predetermined length, measurement of which is completed by an ECG measurement device, is acquired, the computing device (100) may analyze the predetermined length of the acquired data to perform processing. In this case, the processing may be understood as an operation of extracting data that conforms to at least one of the input conditions of the neural network models for ECG reading from the acquired data.

For example, let it be assumed that there exist models for reading different diseases as shown in FIG. 2. In this case, a reading model A (210) and a reading model C (230) may be models that have a 10-second-long single-lead ECG as an input condition. A reading model B (220) and a reading model D (240) may be models that have a 24-hour-long single-lead ECG as an input condition. As such, the matter regarding the length included in the input condition of each model may vary depending on the analysis task determined during the construction and training process of the model. Specifically, the matter regarding the length included in the input condition may vary depending on whether the analysis task of the neural network model corresponds to a disease related to an ECG rhythm characteristic, a disease related to a characteristic other than the ECG rhythm characteristic, or a health condition. The input conditions that vary depending on the analysis task may be summarized as in the following [Table 1].

**[Table 1]**

| **Task Type** | **Sub-type** | **Input Condition** |
|---|---|---|
| Disease related to ECG rhythm characteristic | Arrhythmia (atrial fibrillation, flutter, bradycardia, etc.) | First Period: 24 hours to 72 hours |
| Disease related to characteristic other than ECG rhythm | Heart failure, myocardial infarction, etc. | Second Period: 10 seconds |
| Sleep | Quality of sleep | Third Period: 7 hours to 8 hours |
| Stress | Stress index | - Fourth Period: min. 5 minutes to 24-72 hours |
| | | - Format: Heart Rate Variability (HRV) data |

When single-lead ECG data being measured by an ECG measurement device is acquired in real time, the computing device (100) may continuously check whether the length of the cumulatively stored data corresponds to a length of 10 seconds or a length of 24 hours. When the length of the cumulatively stored data reaches 10 seconds, the computing device (100) may extract a data interval (10) corresponding to 10 seconds and generate it as input data for the reading model A (210) and the reading model C (230). In addition, when the length of the cumulatively stored data reaches 24 hours, the computing device (100) may extract a data interval (20) corresponding to 24 hours and generate it as input data for the reading model B (220) and the reading model D (240). The data generated through such processing may be input to the individual models that match the input conditions. This process may be repeated until the process of acquiring the single-lead ECG data is terminated. When single-lead ECG data having a predetermined length, measurement of which is completed by an ECG measurement device, is acquired, the computing device (100) may identify a 10-second interval and a 24-hour interval from the single-lead ECG data having the predetermined length. The computing device (100) may extract the identified 10-second interval (10) from the predetermined length and generate it as input data for the reading model A (210) and the reading model C (230). In addition, the computing device (100) may extract the identified 24-hour interval (20) from the predetermined length and generate it as input data for the reading model B (220) and the reading model D (240). The data generated through such processing may be input to the individual models that match the input conditions.

When the 10-second-long input data (10) processed from the single-lead ECG data is input to the reading model A (210) and the reading model C (230), the reading model A (210) and the reading model C (230) may each analyze the input data according to a trained task and individually output reading results (31, 33) regarding a disease or a health condition. When the 24-hour-long input data (20) processed from the single-lead ECG data is input to the reading model B (220) and the reading model D (240), the reading model B (220) and the reading model D (240) may each analyze the input data according to a trained task and individually output reading results (32, 34) regarding a disease or a health condition.

Meanwhile, referring to [Table 1], in the case of a model for analyzing stress among the reading models, it may include as an additional input condition that it receives heart rate variability data, not ECG data. Therefore, the computing device (100) may extract single-lead ECG data of a length that satisfies the input condition of the model for analyzing stress from the acquired single-lead ECG data, and may convert the extracted single-lead ECG data into heart rate variability data. The computing device (100) may input the converted heart rate variability data to the model for analyzing stress and calculate a stress index as a reading result.

Through such a processing procedure, the computing device (100) may integrate and manage models with different input conditions into a single system. That is, the computing device (100) may establish an environment for comprehensively and stably diagnosing a disease or a health condition through a data preprocessing operation that considers the input conditions of a plurality of models.

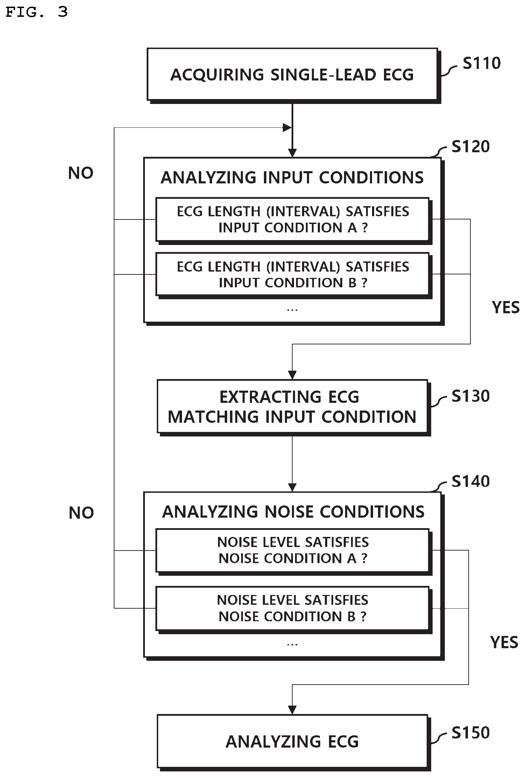

FIG. 3 is a flowchart illustrating a process of analyzing a single-lead ECG according to an embodiment of the present disclosure.

Referring to FIG. 3, a computing device (100) according to an embodiment of the present disclosure may acquire single-lead ECG data (S100). The computing device (100) may acquire the single-lead ECG data through wired or wireless communication with an ECG measurement device. In this case, the computing device (100) may acquire, in real time, the single-lead ECG data being measured by the ECG measurement device. When acquiring the data in real time, the computing device (100) may store the acquired data time-serially. The computing device (100) may also acquire single-lead ECG data having a predetermined length, measurement of which is completed by an ECG measurement device. That is, the computing device (100) may acquire ECG data through communication with an ECG measurement device while the ECG measurement is not completed, and may also acquire ECG data of a specific length in a state where the ECG measurement is completed.

The computing device (100) may analyze at least one of input conditions of a plurality of pre-trained neural network models for reading the single-lead ECG data, based on a length of the single-lead ECG data acquired in step S100 (S120). When a plurality of input conditions exist, the computing device (100) may analyze whether the data acquired through communication with the ECG measurement device satisfies each of the plurality of input conditions. When single-lead ECG data being measured by an ECG measurement device is acquired in real time, the computing device (100) may analyze whether the length of the time-serially stored single-lead ECG data, in the process of being accumulated, satisfies at least one of the input conditions. In this case, the analysis of the input condition (S120) may be repeatedly performed until the real-time acquisition is terminated. When single-lead ECG data having a predetermined length, measurement of which is completed by an ECG measurement device, is acquired, the computing device (100) may identify an interval that satisfies at least one of the input conditions in the single-lead ECG data having the predetermined length.

When the length of the time-serially stored single-lead ECG data satisfies at least one of the input conditions, or when an interval satisfying at least one of the input conditions is identified in the single-lead ECG data having the predetermined length, the computing device (100) may extract ECG data that matches the input condition from the acquired data (S130). The computing device (100) may extract single-lead ECG data of a length that satisfies at least one of the input conditions from the time-serially stored single-lead ECG data. The computing device (100) may extract single-lead ECG data of an interval that satisfies at least one of the input conditions from the single-lead ECG data having the predetermined length.

Meanwhile, when there is an input condition that is not satisfied by the length of the time-serially stored single-lead ECG data, the computing device (100) may perform step S120 again to re-determine whether the input condition is satisfied. As time passes, the length of the single-lead ECG data stored in the computing device (100) will increase, so the computing device (100) may continuously analyze the input conditions by repeatedly performing step S120. In addition, when there is no interval that satisfies any of the input conditions in the single-lead ECG data having the predetermined length, the computing device (100) may perform step S110 again to cumulatively store the single-lead ECG data. The computing device (100) may perform step S120 again based on the cumulatively stored single-lead ECG data to re-identify an interval that satisfies the input condition.

The computing device (100) may analyze a noise level of the single-lead ECG data processed through step S130, and determine whether to input the processed single-lead ECG data to at least one of the plurality of neural network models (S140). An analyzable noise level may exist individually for each of the plurality of neural network models. A noise level individually set for each of the plurality of neural network models may be understood as a noise condition. The computing device (100) may analyze whether the noise level of the single-lead ECG data extracted to match an input condition satisfies a noise condition of a model, among the plurality of models, that has an input condition corresponding to the length of the processed single-lead ECG data. The computing device (100) may determine whether to input the processed single-lead ECG data to the model based on whether the noise condition is satisfied. For example, if the noise level of the data processed according to input condition A does not satisfy noise condition A, the computing device (100) may determine not to input the corresponding data to model A. The computing device (100) may analyze the noise level of other data processed according to input condition A, or perform the process of processing the data according to the input condition again. If the noise level of the data processed according to input condition A satisfies noise condition A, the computing device (100) may determine the corresponding data as input data for model A.

The computing device (100) may perform an ECG analysis by inputting the processed data, determined as input data through step S130, to a model (S150). The computing device (100) may generate a reading result regarding a disease or a health condition through the ECG analysis. Reading results may be generated individually by each of a plurality of models, and a final analysis result may be generated at a time point determined by a user command or at the time point when the ECG measurement is completed. In this case, the computing device (100) may process the reading results generated by each of the plurality of models through a statistical technique or a post-processing neural network model to generate a final analysis result. Therefore, the final analysis result may include a comprehensive diagnosis result for a plurality of result values.

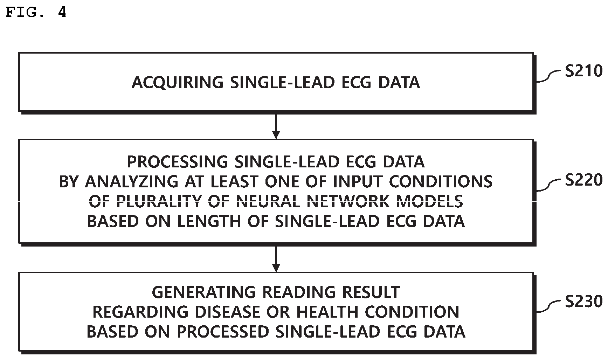

FIG. 4 is a flowchart illustrating a method for analyzing a single-lead ECG using artificial intelligence according to an embodiment of the present disclosure.

Referring to FIG. 4, a computing device (100) according to an embodiment of the present disclosure may acquire single-lead ECG data (S210). The method and cycle of acquiring the single-lead ECG data may vary depending on a user command. For example, if a user wants real-time diagnosis or monitoring, the computing device (100) may reflect the user command and acquire data being measured in real time through communication with an ECG measurement device. If a user wants to check an analysis result at a specific cycle or time, the computing device (100) may communicate with an ECG measurement device at the specific cycle or time to acquire ECG data, measurement of which is completed.

The computing device (100) may process the single-lead ECG data by analyzing at least one of input conditions of a plurality of pre-trained neural network models for reading the single-lead ECG data, based on a length of the single-lead ECG data (S220). The description of step S220 corresponds to the descriptions of steps S120, S130, S140 of FIG. 2 and FIG. 3, and thus will be omitted hereinafter.

The computing device (100) may generate a reading result regarding a disease or a health condition by inputting the single-lead ECG data processed through step S220 to a model, among the plurality of models, that has an input condition corresponding to the length of the single-lead ECG data processed through step S220 (S230).

The various embodiments of the present disclosure described above may be combined with additional embodiments, and may be modified within a scope understandable by a person of ordinary skill in the art in light of the detailed description above. The embodiments of the present disclosure should be understood to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts should be interpreted as being included in the scope of the present disclosure.

## Claims

1. A method for analyzing a single-lead electrocardiogram using artificial intelligence, performed by a computing device including at least one processor, the method comprising:
acquiring single-lead electrocardiogram data; and
processing the acquired single-lead electrocardiogram data by analyzing at least one of input conditions of a plurality of pre-trained neural network models for reading single-lead electrocardiogram data, based on a length of the acquired single-lead electrocardiogram data.

2. The method of claim 1, wherein the acquiring of the single-lead electrocardiogram data comprises:
acquiring, in real time, the single-lead electrocardiogram data being measured by an electrocardiogram measurement device, or acquiring the single-lead electrocardiogram data having a predetermined length, measurement of which is completed by the electrocardiogram measurement device.

3. The method of claim 2, wherein the processing of the acquired single-lead electrocardiogram data comprises:
when the single-lead electrocardiogram data being measured by the electrocardiogram measurement device is acquired in real time, analyzing whether a length of time-serially accumulated single-lead electrocardiogram data satisfies at least one of the input conditions; and
extracting, from the time-serially accumulated single-lead electrocardiogram data, the single-lead electrocardiogram data of a length that satisfies the at least one of the input conditions.

4. The method of claim 2, wherein the processing of the acquired single-lead electrocardiogram data comprises:
when the single-lead electrocardiogram data having the predetermined length, measurement of which is completed by the electrocardiogram measurement device, is acquired, identifying, in the single-lead electrocardiogram data having the predetermined length, an interval that satisfies at least one of the input conditions; and
extracting, from the single-lead electrocardiogram data having the predetermined length, the single-lead electrocardiogram data of the interval that satisfies the at least one of the input conditions.

5. The method of claim 1, further comprising:
determining whether to input the processed single-lead electrocardiogram data to at least one of the plurality of neural network models, by analyzing a noise level of the processed single-lead electrocardiogram data.

6. The method of claim 5, wherein the determining of whether to input the processed single-lead electrocardiogram data to the at least one of the plurality of neural network models comprises:
analyzing whether the noise level of the processed single-lead electrocardiogram data satisfies a noise condition of a model, among the plurality of models, that has an input condition corresponding to the length of the processed single-lead electrocardiogram data; and
determining whether to input the processed single-lead electrocardiogram data to the model having the input condition corresponding to the length of the processed single-lead electrocardiogram data, based on whether the noise condition is satisfied.

7. The method of claim 1, wherein a matter regarding the length of the electrocardiogram data included in the input condition varies depending on an analysis task of the plurality of neural network models.

8. The method of claim 1, wherein:
an input condition of a model for analyzing a disease related to an electrocardiogram rhythm characteristic, among the plurality of neural network models, relates to whether the length of the single-lead electrocardiogram data corresponds to a first time period;
an input condition of a model for analyzing a disease related to a characteristic other than the electrocardiogram rhythm characteristic, among the plurality of neural network models, relates to whether the length of the single-lead electrocardiogram data corresponds to a second time period;
an input condition of a model for analyzing sleep, among the plurality of neural network models, relates to whether the length of the single-lead electrocardiogram data corresponds to a third time period;
an input condition of a model for analyzing stress, among the plurality of neural network models, relates to whether a minimum length of the single-lead electrocardiogram data corresponds to a fourth time period; and
the first time period, the second time period, the third time period, and the fourth time period are different from each other.

9. The method of claim 8, wherein the processing of the acquired single-lead electrocardiogram data comprises:
when the length of the acquired single-lead electrocardiogram data satisfies the input condition of the model for analyzing stress among the plurality of neural network models, extracting, from the acquired single-lead electrocardiogram data, the single-lead electrocardiogram data of a length that satisfies the input condition of the model for analyzing stress, and converting the extracted single-lead electrocardiogram data into heart rate variability data.

10. The method of claim 1, comprising:
generating a reading result regarding a disease or a health condition, by inputting the processed single-lead electrocardiogram data to a model, among the plurality of models, that has an input condition corresponding to the length of the processed single-lead electrocardiogram data.

11. A computer program stored on a computer-readable storage medium, the computer program, when executed by one or more processors, causing operations to be performed for analyzing a single-lead electrocardiogram using artificial intelligence,
the operations comprising:
acquiring single-lead electrocardiogram data; and
processing the acquired single-lead electrocardiogram data by analyzing at least one of input conditions of a plurality of pre-trained neural network models for reading single-lead electrocardiogram data, based on a length of the acquired single-lead electrocardiogram data.

12. A computing device for analyzing a single-lead electrocardiogram using artificial intelligence, the device comprising:
a processor comprising at least one core;
a memory comprising program codes executable by the processor; and
a network unit for acquiring single-lead electrocardiogram data,
wherein the processor is configured to process the acquired single-lead electrocardiogram data by analyzing at least one of input conditions of a plurality of pre-trained neural network models for reading single-lead electrocardiogram data, based on a length of the acquired single-lead electrocardiogram data.
